# EUROPEAN PATENT SPECIFICATION

(11) **EP 1 946 787 B1**
(45) Date of publication and mention of the grant of the patent: **14.05.2014**
(21) Application number: 08250274.1
(22) Date of filing: 22.01.2008
(51) Int. Cl.: A61M 5/178, G09F 3/10, G09F 3/02

(54) **SYRINGE HAVING A SYRINGE LABEL**
SPRITZE MIT EINEM SPRITZENETIKETT
SERINGUE AYANT UNE ETIQUETTE DE SERINGUE

(30) Priority: 22.01.2007 US 897009 P
(43) Date of publication of application: 23.07.2008
(73) Proprietor: Teva Pharmaceutical Industries Ltd, 49131 Petah Tiqva (IL)
(72) Inventor: Lavi-Loebl, Adi, 40600 Tel-Mond (IL); Aharon, Hava, 44374 Kefar Saba (IL); Petter, Ram, 71908 Reut (IL)
(74) Representative: Albutt, Anthony John

(56) References cited:
- EP-A- 0 463 193
- EP-A- 1 536 397
- EP-A- 1 634 816
- WO-A-2004/050038
- US-A- 4 312 523
- US-A- 4 884 827
- US-A- 5 800 893
- US-A- 5 964 736
- US-A1- 2002 038 392
- US-A1- 2003 195 491
- US-A1- 2004 071 917
- US-A1- 2004 168 293
- US-A1- 2004 168 741
- US-A1- 2005 119 622
- US-A1- 2007 031 619
- US-B1- 6 284 339

## Description

### FIELD OF THE INVENTION

The present invention generally relates to a device and method for readily storing and accessing information regarding the contents of a syringe. In particular, an exemplary embodiment of the present invention relates to a syringe and syringe label, which rolls or wraps (these terms are used interchangeably herein) around the syringe and itself.

### BACKGROUND INFORMATION

It is known to adhere a label including graduation markings to a syringe for the purpose of facilitating the administration of a metered dosage. According to draft guidelines for the Committee for Medicinal Product for Human Use of the European Medicines Agency, graduation markings applied in such a manner, however, may be prone to inaccuracies because the labels may become partially detached from the syringe, may shift on the syringe, and/or may be improperly applied to the syringe in the first place. Guidelines drafted by the Committee for Medicinal Product for Human Use of the European Medicines Agency therefore strongly recommend not using an adhered label to place graduation markings on a syringe. "Guideline on the suitability of the graduation of delivery devices for liquid dosage forms," European Medicines Agency, February 18, 2005. One solution to this problem involves embossing or printing the graduation markings directly on the syringe itself. This solution, however, leaves little room on the syringe for communicating required information about the syringe or its contents to the public as required by pharmaceutical regulations.

There is a need, therefore, for a labeling mechanism for syringes that will relate a large amount of information without obscuring important portions of the syringe surface area, while not interfering with syringe use and storage.

It is known to include required information about the syringe or its contents on a label including graduation markings that is adhered to a syringe. This method, however, requires that each syringe have a particularly designed label that rejects the correct graduation markings for that syringe. This poses difficulty in mass production of syringes and syringe labels. Specifically, this method may limit which products a syringe may contain. There is a need, therefore, for a labeling mechanism for syringes that separately labels syringe body characteristics and syringe contents.

WO2004/050038, US 5,964,736, US 2003/0195491 and US 2002/0038392 disclose syringes comprising graduation marks. US 2005/0119622 discloses a colour-coded syringe arrangement for the dispensation of insulin. EP 1536397 discloses labelling for medical devices.

### SUMMARY

Aspects of the invention are set out in the appended claims.

In an exemplary method of the present invention: (i) information may be applied to a label, (ii) adhesive may be applied to inner and/or outer major surfaces of the label, (iii) the label may connected, e.g., permanently connected, to an item, such as a syringe, e.g., on or adjacent one end of the label, and (iv) the label may be rolled about the item and then, if of sufficient length, continued to be rolled about itself and the item.

In an exemplary embodiment of the present invention the label may be at least partially unrolled from the item, for example, so as to allow information on said label to be reviewed by a user of the item. The label may then be re-rolled about the item, for example, so as to restore the label to its initial rolled state. The label may also be adhered to a different surface, such as a vial or medical chart.

An example embodiment of the present invention includes a syringe having a label rolled about the syringe. The syringe may include graduation markings that are permanently denoted, e.g., by etching or embossing the graduations or by forming, such as by molding, the syringe with the graduations or by directly printing the graduations, on the syringe.

In an exemplary embodiment of the present invention, the label is adhered to the syringe by adhesives placed on an inner surface of the label that face the syringe when the label is in a rolled state.

In an exemplary embodiment of the present invention, the label is long enough to ensure that it rolls not only around the syringe but, at least partially, about itself,

In an exemplary embodiment of the present invention, the label is designed to proportionally fit the syringe used. With a particular syringe model, an exemplary embodiment of the label is 80 mm long and 15 mm wide (the width of the label running along the longitudinal axis of the syringe).

In an exemplary embodiment of the present invention, the label, e.g., the inner and/or outer surfaces, may include sufficient surface area to host copious quantities of information, such as drug type, drug strength, pharmaceutical form, expiration date, shelf life data, batch number, warnings, contraindications, instructions for use, patent related information, manufacturer's name, distributor's name, bar codes, etc.

In an exemplary embodiment of the present invention, critical information such as the name of the product, lot number, and expiration date are placed on an outer surface at the far end of the label. In this fashion, such critical information will be displayed on the outer layer of the label, even when the label is wrapped.

In an exemplary embodiment of the present invention, the label is connected to the syringe in an area of the syringe on which there are no graduations or other markings, e.g., along an entire outer circumference of the syringe just below the syringe flange and above the graduation markings.

In an exemplary embodiment of the present invention, the label is wrapped about the syringe above the graduations and below, e.g., 4 mm below, the syringe flange through which the syringe plunger is inserted.

In an exemplary embodiment of the present invention,
the syringe is a Becton Dickinson Hypak SCF1ML 25GA5/81N type syringe. The length of such syringe from needle tip to flange is 62.70 mm, the diameter of the circular portion of the flange is 17.75 mm, and the needle length is 16 mm.

In an exemplary embodiment of the present invention, where the label is rolled just below the syringe flange, the medication in the syringe may be injected by pressing the syringe plunger, e.g., in the standard method using one's thumb towards the syringe flange while the index and middle fingers, positioned directly over and grasping the rolled label, maintain the position of the syringe by resisting the forward force created by the plunger.
The length and thickness of the label may be limited to assure that the radial thickness of the ring created by the label when rolled about the syringe allows for adequate finger space under the flange to, for example, comfortably resist the forward movement of the syringe while the plunger is being depressed.

An exemplary method according to the present invention involves communicating information to a syringe user. The syringe may include a flange and a barrel having graduation markings running along a longitudinal axis of the barrel. If the label does not have a transparent window or portion, the label is connected to the barrel and positioned so as to not cover the graduation markings, e.g., between the flange and the graduation markings. The label may also have a length exceeding a circumference of the barrel. The label is then wrapped around the barrel and itself. An adhesive property of at least a portion of the bottom surface of the label maintains the label in a wrapped state.

As indicated above, the thickness and length of the label may be chosen such that a circumference of the label wrapped around the barrel is sufficiently smaller than a circumference of the flange so as to allow a user to grasp the barrel over the wrapped label between, e.g., the index and middle fingers, without slipping over the flange while using the syringe. To the extent the flange is not circular, the circumference corresponds to that of the smallest circle fitting around the flange.

In an exemplary embodiment of the present invention, the label may be unrolled for the purpose of reviewing data stored on its outer and/or inner surfaces.

The label may have a shape memory or be processed, e.g., using a heating iron, such as a curling iron, so as to be biased into the rolled state. Such property or processing of the label may be useful to facilitate a manual re-rolling of the label around the syringe or to provide for an automatic re-rolling, which would prevent the label from dangling from the syringe when not being read.

In an exemplary embodiment of the present invention, the label may have an adhesive and/or may have cling properties to assure that it remains rolled about the syringe and itself until a user wants to read the label.

In an exemplary embodiment of the present invention, the label itself may be made from an electret material, i.e., a material which carries a permanent electric charge, which will cling to most clean surfaces. An electret label may be created, for example, by passing the label material through an electric field at an elevated temperature and then cooling the label material before the induced electric dipole moment generated by the field has time to dissipate. See, for example, U.S. Patent No. 6,284,339.

In an exemplary embodiment of the present invention, the label may include an adhesive along an inner surface facing the syringe when in a rolled state. The adhesive may be of a sort that does not leave residue on contact surfaces, e.g., on the outer non-adhesive surface of the label. For example, the adhesive may be a S692N clear acrylic based adhesive.

In an exemplary embodiment of the present invention, the label may be a Fasson PP NG TOP White / S692N / BG42.

In an exemplary embodiment of the present invention, the label may be made from an electret material and also include adhesive, e.g., at predetermined portions of the label.

The label and adhesive may be designed to function well and remain stable under both room and cool operating conditions, e.g., two to eight degrees Celsius.

In an exemplary embodiment of the present invention, an end of the label may be connected to the syringe using an adhesive (which may be the same or different than the adhesive used on other portions of the inner surface of the label) or via other known means for connecting a label to a surface, e.g., welding, stapling, etc. The end of the label may be permanently fixed to the syringe or removable from the syringe.

In an exemplary embodiment of the present invention, the adhesive on the label may be applied to the entire inner surface of the label or to select portions of the inner surface.

The application of the adhesive, including the type of adhesive, the thickness of the adhesive layer, and the placement of the adhesive on the inner surface of the label, may be specifically designed to control the manner in which the label unrolls and re-rolls onto the syringe.

Different patterns of the adhesive application may affect, for example, the amount of precision required to neatly re-roll the label about the syringe and itself without, for example, creating air bubbles, the overall thickness of the rolled label (a tightly rolled labeled will have a lower rolled thickness), the amount of force required to unroll the label, the ease with which the label may be re-rolled, etc.

In an exemplary embodiment of the present invention, the entire inner surface of the label may be coated with adhesive but the amount of adhesive on the label may increase or decrease along the length of the label.

In an exemplary embodiment of the present invention, portions of the inner surface of the label may be coated with adhesive and adhesive on some of these portions may be deadened, i.e., rendered non-adhesive, for example, by applying a coating, e.g., a varnish coating, over the adhesive and/or exposing these portions of the adhesive to conditions which are known to render the adhesive non-adhesive.

In an exemplary embodiment of the present invention, dots or strips, e.g., transverse strips, of adhesive may line the label and the spacing of these dots or strips may vary along the length of the label.

In an exemplary embodiment of the present invention, the strips and/or dots may be placed, for example, between separate sections of information included on the label such that upon unrolling the label a user encounters an increased resistance to unrolling upon the start of each new section of label information. The strips and/or dots of adhesive prevent the label from uncontrollable further unrolling (thereby forcing the user to unnecessarily hold the entire length of the label) while the relevant section of the label is being read.

In an exemplary embodiment of the present invention, the free or exposed end of the label may be designed to enhance its gripability and/or to facilitate the unrolling of the label. For example, (i) the portion of the label adjacent the free end, e.g., the last 5mm of the label, may be designed thicker, (ii) a finger hold, e.g., a small ring or raised flap, may be connected to or adjacent the free end of the label, (iii) the outer surface of the label, e.g., adjacent its free end, may have a raised texture, and (iv) the free end of the label may include one or more points, e.g., a saw tooth profile.

In another exemplary embodiment, the inner surface of the label adjacent the free end of the label may include a strip of stronger adhesive (or a strip of thicker coating of the same adhesive as on the rest of the inner surface). This adhesive strip (and the relatively weaker adhesive between the strip and free end of the label) maintains the label in the rolled state but at the same time assures that a user does not have difficultly unrolling or peeling the free end of the label off the syringe or label itself.

In an exemplary embodiment of the present invention, multiple labels may be rolled around the syringe and each other. The labels may vary in color and/or length such they are easily distinguishable and their ends (opposite the ends connected to the syringe) are offset in the wrapped state. Multiple labels may be used, for example, in a situation where it is preferred not to have a single longer label, which may be more difficult to re-roll around the syringe. Multiple labels may also be useful to separate and thus better organize the information on the labels. For example, a red tinged label may be used for contraindications, a green tinged label may be used to carry instructions for use, etc.

A syringe according to an exemplary embodiment of the present invention includes a barrel and a label. The barrel has graduation markings running along a longitudinal axis of the barrel configured to facilitate the administration of a metered dosage. The label has two or more strips each having a different length, a top surface, and a bottom surface facing the barrel when the label is wrapped around the barrel. At least a portion of the bottom surface of each strip exhibits an adhesive property. A width of each strip extends along a longitudinal axis of the barrel and the length of each strip extends transverse to the longitudinal axis. A first of the strips is connected on a first end to the barrel. The adhesive property of the bottom surface of the first strip maintains the first strip in a wrapped state when the first strip is wrapped around the barrel but allows the first strip to be at least partially unwrapped from the barrel upon application of a first predetermined force to the first strip in a direction away from the barrel. A second of the strips is shorter in length than the first strip. A first end of the second strip is connected to the outer surface of the first strip. The adhesive property of the bottom surface of the second strip maintains the second strip in a wrapped state about both the barrel and the first strip when the first and second strips are wrapped about the barrel but allows the second trip to be at least partially peeled off from the first strip upon application of a second predetermined force to the second strip in a direction away from the first strip. At least the inner surface of both the first and second strips may contain indicia.

The label may also include, for example, a third strip having a length shorter than the second strip and connected on a first end to the outer surface of the second strip. At least the inner surface of the third strip may have indicia thereon.

So as to assure that an unwrapping of an outer strip, e.g., the third strip, does not inadvertently unwrap another strip along with it, e.g., the second strip (which might happen if the third strip is only slightly shorter than the second strip), each strip can be configured to demonstrate a different level of adhesion. For example, the third strip may be configured to demonstrate less adhesion than the second strip and the second strip configured to demonstrate less adhesion than the first strip.

In an exemplary embodiment of the present invention, a wider label may be used which is folded, for example, along its length prior to being wrapped about the syringe. Upon unwrapping, the label may be unfolded by a user by pulling a portion of the label along the longitudinal axis of the syringe barrel so as to reveal information on portions of the label, which are not exposed when the label is folded and/or wrapped about the syringe. The label may be partially cut along the length of the syringe so as to allow portions of the label between the free end and the cut to be unfolded while only unwrapping a portion of the label.

In an exemplary embodiment of the present invention, a label with a transparent window may be wrapped once or multiple times around the syringe body. The label may extend partially or entirely over the graduation markings on the syringe (which may be permanently marked on the syringe) but is wrapped or applied to the syringe such that the transparent window portion of the label is over the graduation markings so as to not obscure such markings. The window may occupy, for example, a rectangular strip portion of the label running along the length of the syringe. Multiple windows may be provided to expose graduation markings along the entire circumference of the syringe.

In an exemplary embodiment of the present invention, a transparent label, e.g., with high relative opacity, or a translucent label may be wrapped once or multiple times around the syringe body. The limited transparency of the label will permit an observer to easily view the outer layer of the label. Simultaneously, a dedicated observer will be capable of using the limited transparency to view information lying behind the outer layer of the label.

A syringe according to an exemplary embodiment of the present invention includes a barrel and a label. The barrel has graduation markings running along a longitudinal axis of the barrel configured to facilitate the administration of a metered dosage. The label has a width and a length and is connected to the barrel at a first end of the label such that the width of the label extends along the longitudinal axis. The label has a top surface and a bottom surface opposite the top surface facing the barrel when the label is wrapped around the barrel. At least a portion of the bottom surface of the label exhibits an adhesive property allowing the label to remain in at least a partially wrapped stated around the barrel. The label includes at least one transparent window extending along the width of the label and the longitudinal axis of the barrel. The label is positioned on the barrel such that the window extends over at least a portion of the graduation markings when the label is wrapped and allows a user to read the graduation markings without unwrapping the label. The window may be more transparent than a remainder of the label. Alternatively, the entire label may be transparent and have indicia except for the window, which may be transparent but devoid of indicia, so as to allow visualization of the graduation markings.

The window may be elongate and cover an entire length of the graduation markings.

An example embodiment of the present invention is described in more detail below with reference to the appended Figures. The foregoing description and examples have been set forth as mere illustrations and are not intended to be limiting. Each of the disclosed aspects and embodiments may be considered individually or in combination with other aspects, embodiments, and variations thereof. The steps of the methods described herein are not confined to any particular order of performance.

### BRIEF DESCRIPTION OF THE DRAWINGS

Figure 1 is a plan view of a syringe according to an exemplary embodiment of the present invention with a label in a rolled or wrapped state.
Figure 2 is a plan view of the syringe of Figure 1 with the label in an unrolled or unwrapped state.
Figure 2A is a perspective view of the syringe of Figure 1 grasped by a user prior to use.
Figure 3 is a plan view of an outer surface of the label of Figures 1 and 2 shown completely unrolled and detached from the syringe.
Figures 4A-4L are plan views of the inner surface of the label of Figures 1 and 2 shown completely unrolled, detached from the syringe, and having different exemplary adhesive application patterns.
Figure 5 is a plan view of an exemplary embodiment of a syringe according the present invention with multiple wrapped labels.
Figure 6 is a plan view of the syringe of Figure 5 with the labels in an unrolled or unwrapped state.
Figure 7 is a plan view of the syringe of Figure 5 with the labels in an unrolled or unwrapped state and with a perspective view of the shorter label partially peeled off the longer label.
Figure 8 is a plan view of a label according to an exemplary embodiment of the present invention.
Figure 9 is a plan view of an exemplary syringe according to the present invention with the label of Figure 8 wrapped around it.
Figure 10 is a plan view of an exemplary embodiment of a folded label according to the present invention.
Figure 10a is a plan view of the label of Figure 10 in an unfolded state.
Figure 10b is a transverse cross sectional view of the label of Figure 10 taken along lines 10b-10b.
Figure 11 is a side view of an exemplary label according to the present invention with a thicker more readily graspable free end.
Figure 12 is a side view of an exemplary label according to the present invention with a thicker more readily graspable free end.
Figure 13 is a plan view of an exemplary label according to the present invention with a saw tooth profile free end.
Figure 14 is a plan view of an exemplary label according to the present invention with a pointed profile free end.
Figure 15 is a plan view of an exemplary label according to the present invention with a ring tab free end.
Figure 16 is a plan view of a syringe according to an exemplary embodiment of the present invention with a label containing a transparent section in a rolled or wrapped state.
Figure 17 is a plan view of the syringe of Figure 16 with the label containing a transparent section in a partially unrolled or unwrapped state.
Figure 18 is a plan view of a syringe according to an exemplary embodiment of the present invention with a label containing a transparent or semi-transparent section in a rolled or wrapped state.
Figure 19 is a plan view of the label illustrated in Figure 18 shown unrolled or unwrapped and separated from the syringe.

### DETAILED DESCRIPTION

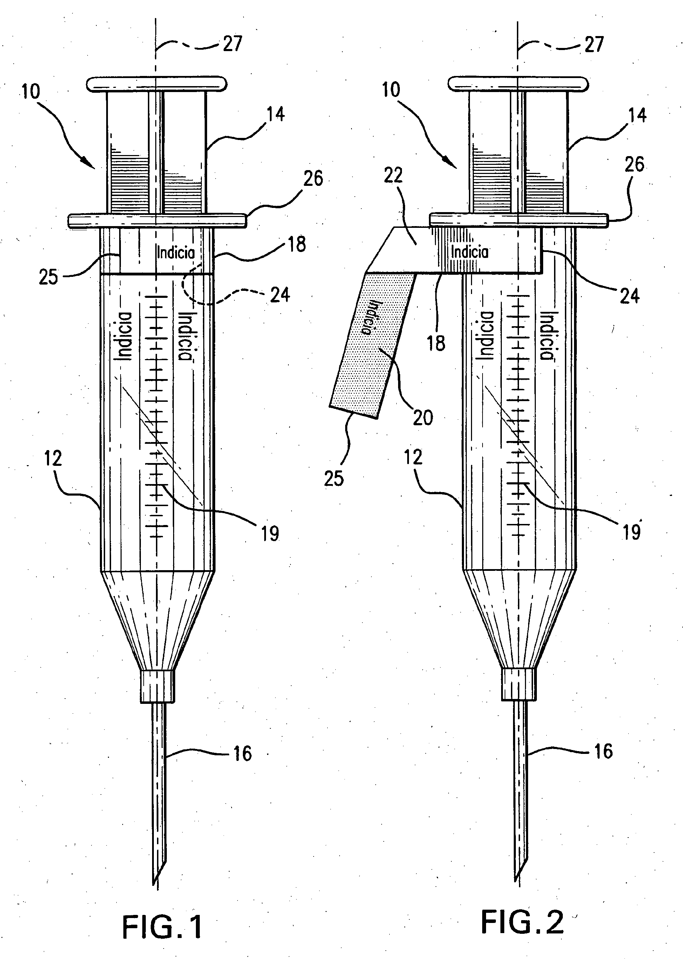

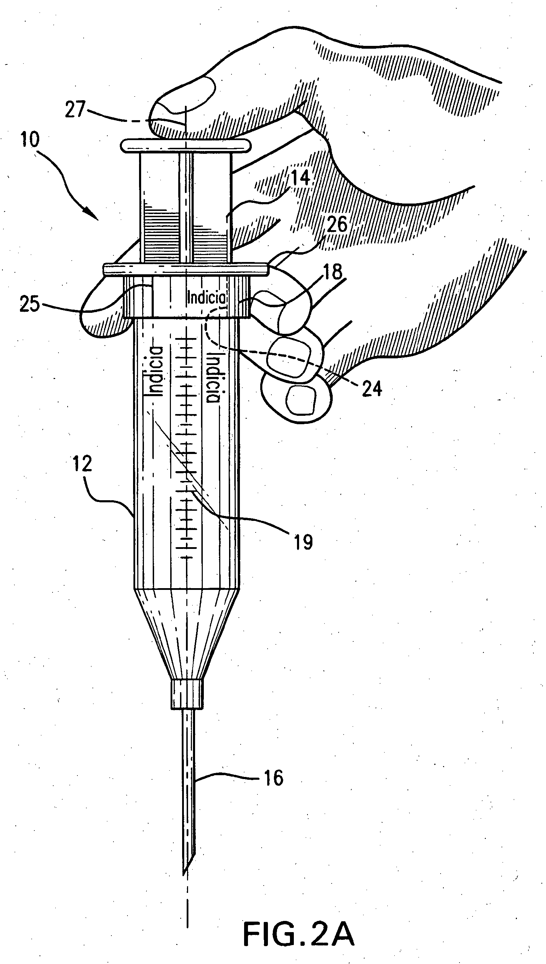

A syringe 10 according to an exemplary embodiment of the present invention is illustrated schematically in Figure 1. The syringe 10 includes a syringe body 12, a plunger 14 slidingly disposed in the syringe body 12, a needle 16 extending from a distal end of the syringe body 12, and a label 18 rolled about the syringe body 12 and itself. The syringe body includes graduations 19, which may be applied to the syringe body 12 via a label or may be permanently denoted in the syringe body, e.g., via etching, embossing, or molding the syringe body 12 with the graduation in the wall of the syringe body 12. The syringe body 12 also includes indicia (labeled "Indicia"), such as numbering for the graduation, which leaves little free surface area on the syringe body 12 for communicating information to the user of the syringe 10. Label 18 serves this role. The label 18 is shown unrolled from the syringe body 12 in Figure 2.

Label 18 may, for example, be a Fasson PP NG TOP White / S692N / BG42, which is substantially stable when exposed to cold temperatures of 2° to 8° Celsius and includes a facestock of bi-axially oriented, glossy polypropylene film (with a print-receptive top coating) having a basis weight of 45 g/m2 (ISO 536) and a caliper of 0.060 mm (ISO 534). Label 18 may also include a laminate with a total caliper of 0.131 and a liner made of BG42 supercalendered glassine paper with a basis weight of 65 g/m2 (ISO 536), a caliper of 0.057 mm (ISO 534), and a transparency of 46% (ISO 534).

Label 18 may have information (labeled "Indicia") on its inner surface 20 and/or its outer surface 22. The label 18 is shown connected to the syringe body 12 just below the flange 26 of syringe body 12, e.g., 4 mm below the flange 26. Label 18 may also be connected to the syringe body 12, for example, at other indicia free locations. An end 24 of the label 18 is shown as a dashed line in Figure 1 as it is covered by other portions of the label 18 when the label 18 is wrapped upon itself. The label 18 is shown wrapped upon itself for about a quarter of its length. However, longer labels 18 may be used such that the label wraps around the syringe and itself multiple times.

As illustrated in Figure 2A, in order to use syringe 10, a user, for example, places the portion of the syringe body 12 with the label 18 rolled about it between his or her index and middle fingers and uses his or her thumb to advance the plunger 14 into the syringe body 12. While the ring formed by the rolled label 18 over the syringe body 12 raises the user's fingers higher over the syringe body surface than they would be otherwise, the flange 26 is sufficiently large to ensure that a user, e.g., the average user, can comfortably restrain forward movement of the syringe during depression of the plunger 14, i.e., the label and flange 26 are designed to ensure that the user's fingers do not slip past the flange during depression of the plunger 14. Alternatively, a user can hold the syringe body 12 below the label 18, preferably at a portion of the syringe body 12 not including indicia or graduation markings 19.

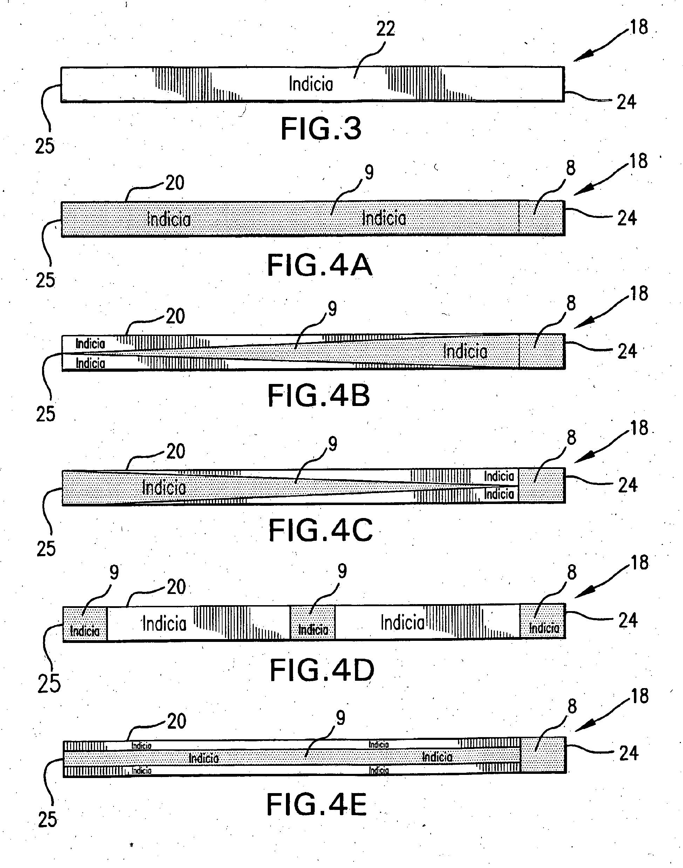

Figure 3 illustrates an outer surface 22 of the label 18 which is free of adhesive. In the wrapped state illustrated in Figure 1, only a portion of the outer surface 22 extending around the circumference of the syringe body 12 is exposed and not rolled upon itself. This section of the label 18 may optionally be reserved for information intended to be communicated and relevant to every user, such as the name of the medication contained in the syringe, lot number, expiration date, etc. The remaining length of the label 18 is not exposed when the label is rolled because the label 18 is rolled upon itself thus concealing information on the remaining portion of the label 18. In order to access this additional information (which may be on either side of the label 18) a user must unroll the label 18 by either rotating the syringe 10 while holding exposed end 25 of label 18 steady or by holding the syringe 10 steady and unwinding the label 18 about the syringe 10.

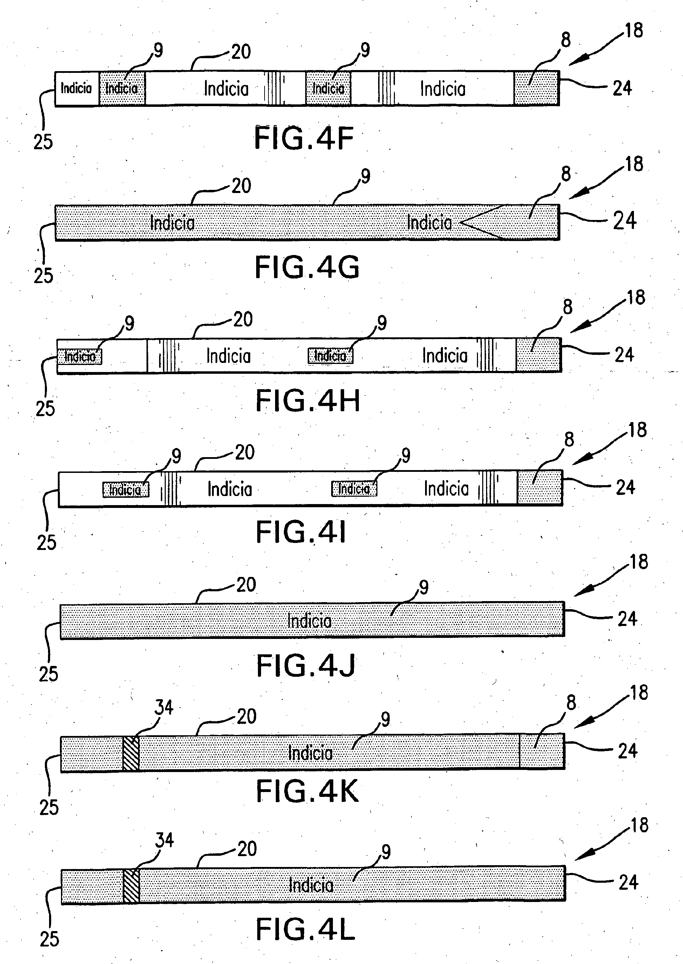

The resistance to unrolling the label 18 will vary depending on the type, amount, and distribution of adhesive 9 on the inner surface 20 of the label 18. Various adhesive distribution patterns are illustrated in Figures 4A-4L, which provide for a different feel when rolling and unrolling the label 18. In all of the embodiments illustrated in Figures 4A to 4I and 4K, a permanent adhesive 8 or other known technique for connecting a label, such as welding, stapling, etc. is used to permanently secure the label 18 to the syringe body 12 at the end 24 of label 18. However, each label 18 illustrated in Figures 4A-4I and 4K may also be applied to the syringe body 12 non-permanently, i.e., without permanent adhesive 8 (adhesive 9 may be applied to the area shown coated by adhesive 8), thus allowing a user to fully remove the label 18, e.g., for record keeping purposes.

In Figure 4A, a non-permanent adhesive 9 is used to removably secure the label 18 to the syringe body 12 and to the label 18 itself when rolled upon itself. Adhesive 9 is applied to the entire inner surface 20 of the label 18 other than the portion coated with adhesive 8. The thickness of the adhesive coating may taper towards 25, so as to reduce resistance as the label 18 is unrolled. This may be useful to facilitate unrolling of the label 18 once the user has expressed a clear desire to gain access to extended portions of the label 18. The thickness of the adhesive coating may also increase towards end 25, so as to increase the resistance to unrolling as the label 18 is unrolled, which may be useful to preventing unrolling of an unintentionally large amount of the label in one stroke or tug on the label 18.

In Figure 4B the permanent adhesive 8 has an increasing triangular section so as to increase removal resistance towards the end 24 of the label 18. This increased resistance provides feedback to a user and, in effect, provides a warning that the end 24 of the label 18 has been reached and that further pulling may remove the label 18 completely from the syringe 10. The adhesive 8 does not necessarily permanently adhere the label 18 to the syringe; it merely holds the label more strongly than adhesive 9.

In Figure 4C the label 18 includes an increasing triangular section of the adhesive 9 used to affix the label 18 in a rolled state to the syringe body 12 and itself. This embodiment provides for reduced resistance to unrolling as the label 18 is unrolled, facilitating unrolling of the label 18 once the user has expressed a clear desire to gain access to extended portions of the label 18.

In Figures 4D, 4F, 4H, and 4I a series of non-contiguous sections of non-permanent adhesive 9 are used to affix the label 18 in a rolled stated to the syringe body 12 and itself. This embodiment facilitates and allows the unrolling of limited portions of the label 18 between the sections of adhesive while preventing the label 18 from completely unrolling beyond the adhesive section without additional tension applied by the user.

In Figures 4F and 4I, the first non-contiguous section of non-permanent adhesive 9 is spaced away from the end 24 of the label 18 so to provide the user with a non-adhered finger hold label section which may facilitate the initial unrolling of the label 18.

In Figure 4J the entire inner surface 20 is coated with non-permanent adhesive 9 allowing the user to completely remove and replace label 18 at will.

In Figure 4G the permanent adhesive 8 has an increasing triangular section so as to increase removal resistance towards the end 24 of the label 18. This increased resistance provides feedback to a user and, in effect, provides a warning that the end of the label 18 has been reached and that further pulling may remove the label 18 completely from the syringe 10.

In Figures 4K and 4L the inner surface 20 of the label 18 is coated with a first adhesive 9 but also includes a strip 34 of stronger adhesive (or a strip of thicker coating of the same adhesive 9 as on the rest of the inner surface 20) adjacent but spaced away from the free end 25 of the label 18. This adhesive strip 34 (and the relatively weaker adhesive between the strip and free end 25 of the label 18) maintains the label 18 in the rolled state but at the same time assures that a user does not have difficultly unwinding or peeling the free end 25 of the label 18 off the syringe 10 or label 18 itself.

Adhesive 9 may, for example, be a S692N adhesive, which is substantially stabile when exposed to cold temperatures of 2° to 8° Celsius, acrylic based, leaves little residue upon removal of the label 18, has excellent UV resistance and weatherability, has good adhesion and re-adhesion performance, and can be used in direct contact with dry and moist non-fatty foodstuffs. This adhesive has an application temperature of 5° Celsius and a service temperature of -20° to 80° Celsius.

The portions of the label 18 in Figures 4A to 4L outside the areas marked as including adhesive 9 may either not include adhesive 9 at all or may be deadened, i.e., may include adhesive which has been treated, e.g., using a varnish, to eliminate or reduce the adhesive properties of these sections of the label 18.

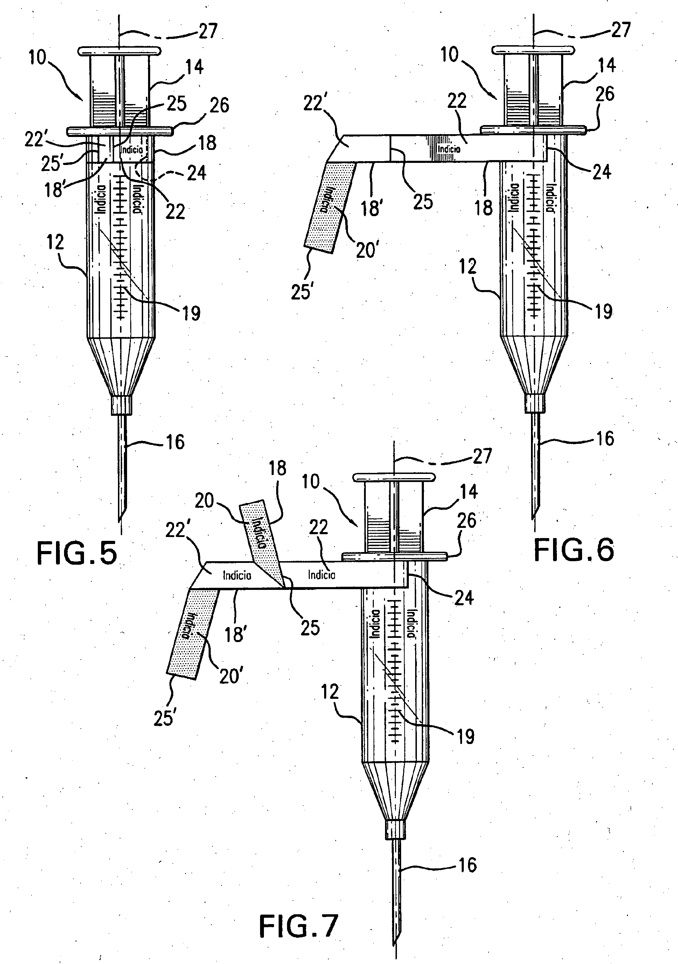

In an exemplary embodiment illustrated in Figures 5 to 7, multiple labels 18, 18', for example, of different lengths and/or colors, can be rolled about the syringe 10. The multiple labels 18, 18' provide for a practical means to communicate additional information to a user (one longer label may become unwieldy) and to organize this information in a clear and straightforward manner. Only two labels 18, 18' are shown in Figures 5 to 7 but additional labels, e.g., of different lengths, may also be used. Each label 18, 18' may contain different information relating to the medication and/or syringe. For example, the longer label 18' may be colored or tinted red and contain contraindications for the drug and the shorter label 18 may be colored or tinted green and include instructions for use.

The labels 18, 18' are illustrated unrolled in Figure 6. In this state the entire length of the outer surface 22 of label 18 and the entire length of the inner surface 20' of label 18' is exposed, and a portion of the outer surface 22' of label 18' (that which is not covered by label 18) is exposed. If a user would like to read more information, for example, about contraindications, he or she may peel label 18 off of label 18' so as to reveal the portion of the outer surface 22' of label 18' covered by label 18, as illustrated in Figure 7. The inner surfaces 20, 20' of the labels 18, 18' may be coated with adhesive 9, e.g., in any of the patterns illustrated in Figures 4A-4L. Further, the end 24 of label 18 may be permanently or removably adhered to the label 18' (or to the syringe body 12 adjacent to where the label 18 is adhered to the syringe body 12) using adhesives 8 or 9, respectively.

The labels in all of the embodiments described above may be made from an electret material, i.e., a material which carries a permanent electric charge, which will cling to most clean surfaces. An electret label may be created, for example, by passing the label material through an electric field at an elevated temperature and then cooling the label material before the induced electric dipole moment generated by the field has time to dissipate.

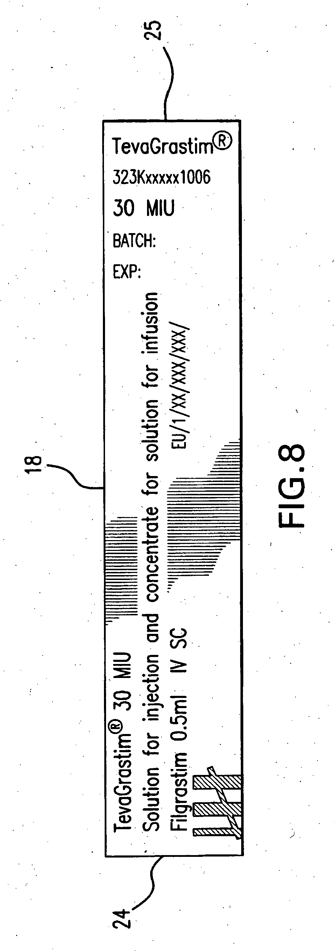

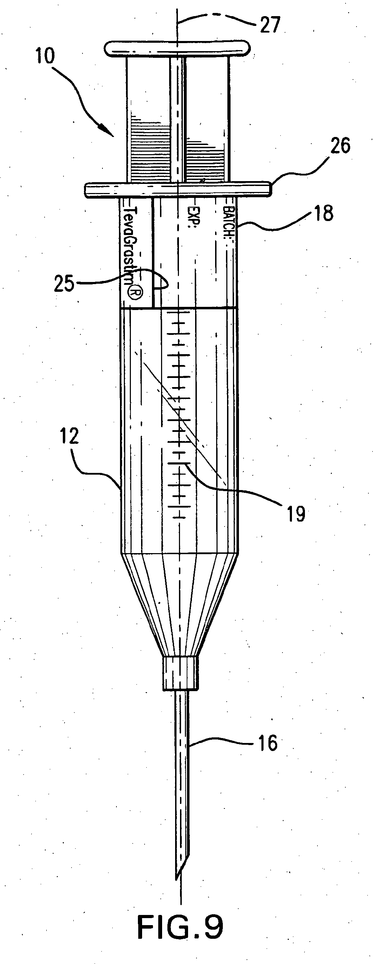

Figure 8 is a plan view of an exemplary embodiment of the label 18 of the present invention. Label 18 is wrapped around the syringe 10 in Figure 9. Text on the portion of the label 18 that is concealed when the label 18 is wrapped about itself runs along the length of the label 18 (transverse to the longitudinal axis of the syringe 10). Text on the portion of the label 18 that is visible even when the label 18 is wrapped around the syringe 10 runs transverse to the longer axis of the label 18, i.e., along the length of the syringe 10. Critical data, such as expiration date, drug name, dosage, etc., which an average user may need to review before using the syringe 10, may be printed in this exposed portion. This arrangement assures that the indicia is aligned in the direction most convenient to the user and, therefore, facilitates reading of the label 18.

The information contained in the portion of the label concealed when the label 18 is wrapped may be broken down into distinct sections. The section closest to the free end 25 (and, therefore, most readily reachable by unrolling the label 18) may contain information more frequently accessed by the average user of the syringe 10 or contain information more critical to the average user, e.g., drug type, expiration date, drug dosage and strength, etc. (this may be redundant to the transversely aligned information contained on the exposed portion of the label 18 but may also include additional detail). Information less critical or less frequently accessed by the average user of the syringe 10 may be printed on sections of the label 18 closer to the end 24 of the label 18. The various sections of information may be separated by transverse lines. Alternatively, different font or different color font may be used so as to allow a user to distinguish the beginning and end of each section on the label 18. A stronger or different adhesive may also be applied between these sections to prevent the label from unrolling while the relevant section is being read.

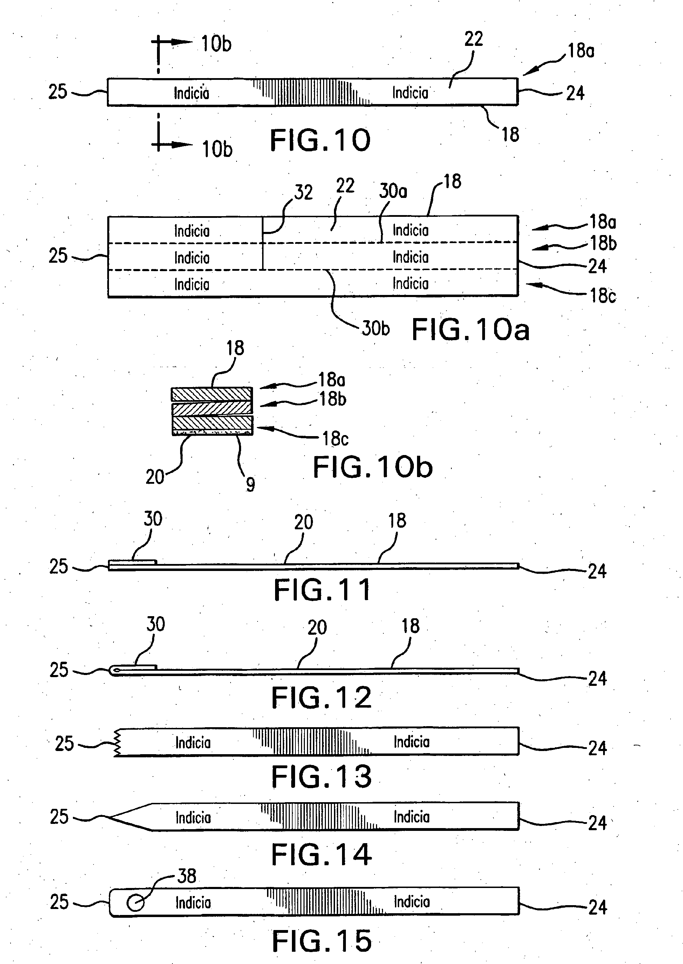

Figure 10 illustrates an exemplary embodiment of a folded label 18 of the present invention in plan view. The label is shown detached from the syringe 10 but may be wrapped about the syringe 10 similar to the label in Figure 1. Figure 10a shows the label 18 in an unfolded state. The label 18 is folded lengthwise along dashed lines 30a and 30b prior to being wrapped about the syringe body 12. Upon unwrapping, the label 18 may be unfolded by a user to reveal information on portions of the label 18 which are not exposed when the label is folded and wrapped about the syringe 10. This embodiment allows for a label much wider (along the length of the syringe 10) than that used in the embodiments of Figures 1 to 9 and, therefore, much wider than any available space on the syringe body 12. The label 18 may be partially cut along the length of the syringe 10, for example, along line 32, so as to allow portions of the label 18 between the free end 25 and the cut line 32 to be unfolded while only unwrapping a portion of the label 18.

A transverse cross section of the label 18 taken along lines 10b-10b in Figure 10 is shown in Figure 10b. The label 18 is folded along fold lines 30a and 30b so as to divide the label 18 into three sections 18a, 18b, and 18c, which can be seen folded and stacked in Figure 10b. As shown in Figure 10b only the bottom surface 20 of section 18c has adhesive 9 applied. Adhesive 9 can be applied to the entirety or portions of the surface 20, e.g., according to any of the patterns illustrated in Figures 4A-4L. Sections 18a, 18b, and/or 18c may have adhesive on either side to maintain the label 18 in the folded state. Alternatively, piece of tape (not shown) on end 25 may be used to prevent label 18 from partially unfolding while wrapped about the syringe 10.

In all of the embodiments of the label 18 disclosed herein, the label 18 may include a non-adhesive coating, e.g., a silicone coating, on its outer surface 22 so as to facilitate unwrapping of the label 18. Further, the label 18 may include one or more transverse weakened sections, e.g., score marks, which allow a user to remove a portion of the label 18, e.g., for record keeping purposes or to adhere to a medical container or a patient's chart, etc.

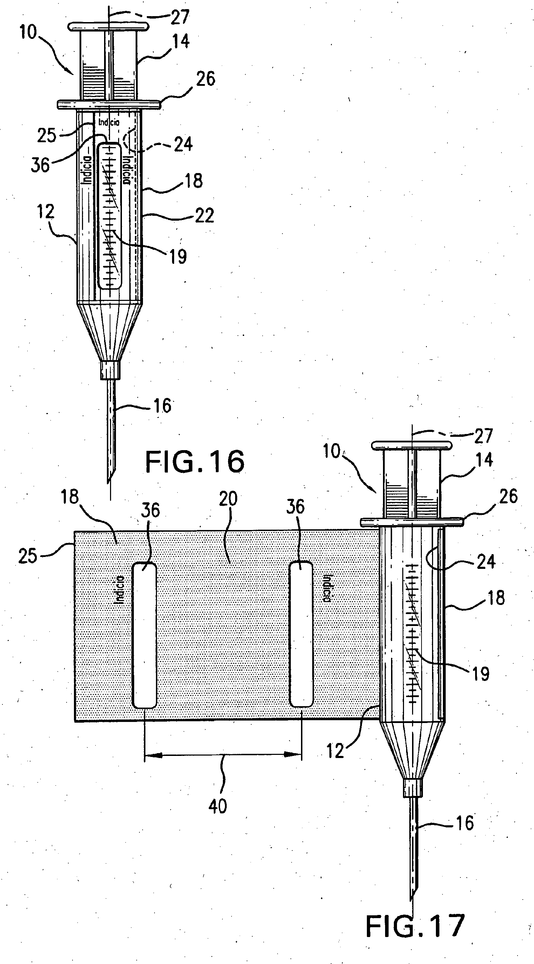

In an exemplary embodiment of the present invention, the free or exposed end 25 of the label 18 may be designed to enhance its gripability and/or to facilitate the unrolling of the label. For example, as illustrated in Figure 11, the portion of the label 18 adjacent the free end 25, e.g., the last 5 mm of the label, may be designed thicker by attaching, e.g., via adhesive, a second layer 30 of the label 18 to the free end 25 of the label 18. Alternatively, as illustrated in Figure 12, the free end 25 may be folded and connected, e.g., via adhesive, to itself or, as illustrated in Figure 15, may include a ring tab, i.e., a rounded profile and a hole 38 in the free end 25 of the label. The free end 25 of the label 18 may also have a saw toothed or pointed end, as illustrated in Figures 13 and 14, respectively, to facilitate unpeeling of the free end 25 of the label 18. These designs may be used in conjunction with adhesive application designs, e.g., as depicted in Figures 4A-4L, and/or with window or transparency designs, e.g., as depicted in Figures 16-19.

As depicted in Figure 16, in an exemplary embodiment of the present invention a label 18 which is wrapped about a syringe body 12 may contain or consist of a transparent or semi-transparent window or series of windows 36 which permits observers to view indicia from the syringe body 12 or from otherwise concealed layers of the label 18 without unwrapping the label 18. The window or windows 36 may permit viewing of indicia on the syringe body 12, e.g., graduation markings 19 (which may be etched, embossed, or otherwise permanently marked on the syringe body 12), in order to make full use of data on the syringe body 12 without unnecessarily unwrapping or removing the label 18 from the syringe 10.
The outer surface 22 of the label 18 may have a roughened texture or ribs to facilitate use of the syringe 10, e.g., prevent slippage of the syringe 10 between the user's fingers during injection, while the label 18 is in a wrapped state.

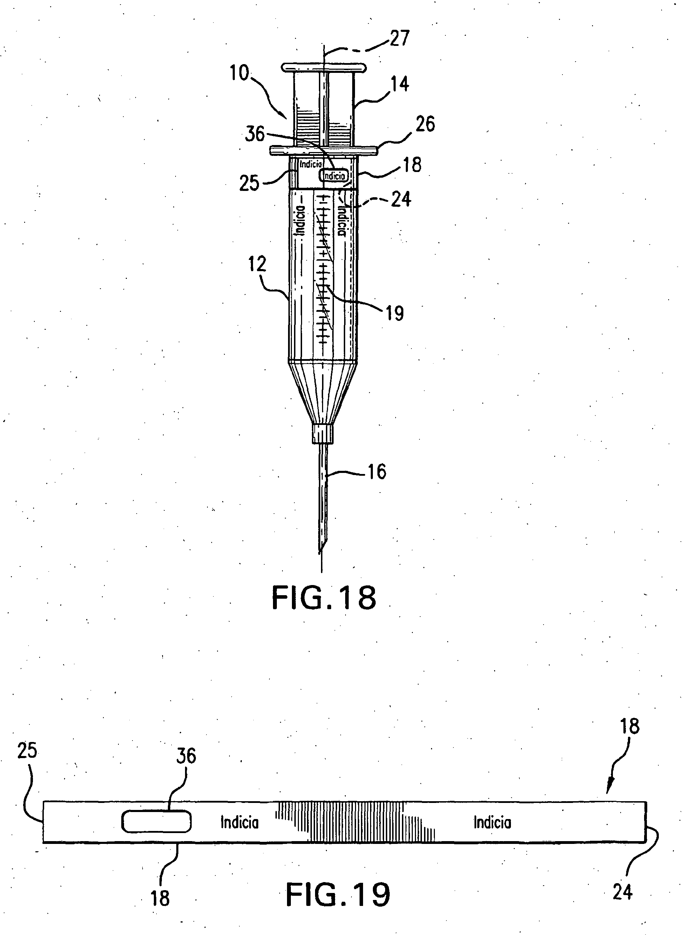

As depicted in Figure 17, which shows the label 18 of Figure 16 partially unrolled, a series of windows 36, e.g., extending in a direction along the length of the syringe body 12 (Figure 16 and 17) or circumferentially (Figures 18 and 19), on the label 18 may be separated by a specified distance 40 matching the windows 36 with informative indicia on the syringe 10 or label 18 itself, e.g., the graduation markings 19, that would otherwise be concealed. For example, the distance 40 between windows 36 might be the circumference of the syringe body 12, in which case the windows 36 would line up with one another, permitting a user to view the graduation markings 19 on the syringe body 12 while the label 18 is wrapped around the syringe body 12. In another example, the distance 40 between windows 36 might be half the circumference of the syringe body 12, which would align the windows 36 to the front and back of the syringe 10 when the label is rolled about the syringe body 12, permitting a user to view indicia, e.g., graduation markings 19, on either side of the syringe body 12. The features of label 18 as depicted in Figures 16-19 maybe used in combination with in any of the embodiments described herein. In an exemplary embodiment, however, adhesives 8 and/or 9 would not extend over the window 36 so as to assure that the view through the window 36 is not obscured. In another exemplary embodiment, indicia may be printed on the window 36. This may be useful to supplement indicia on the syringe body 12 visible through the window. For example, if the volume units associated with the graduation markings 19 on the syringe body 12 are in milliliters (ml) indicia printed on the window 36 may include an alternate set of units more familiar to a given user, e.g., cubic centimeters (cc), or other supplementary information.

Figure 19 illustrates a plan view of a label 18 with a single window 36. The window extends circumferentially when wrapped around the syringe 10, as illustrated in Figure 18. The window or windows 36 may permit the average user to view indicia that is on a concealed layer of the label 18. Viewing otherwise concealed indicia may be particularly useful where indicia that would otherwise be duplicated on the label 18 is positioned in such a fashion that one instance of the indicia appears through the window 36 when the label 18 is wrapped, and appears on the label 18 near the end 24 when the label is unwrapped.

In an exemplary embodiment, multiple windows 36 or alternatively sized windows 36 could be set in the label 18. It is possible for the entirety of the label 18 to be transparent or semi-transparent, effectively making the entirety of the label 18 a window in order to maximize a user's ability to view indicia that would otherwise be concealed by the label 18.

The window feature generally is also useful when, for example, after reading the label, it is intended to adhere the label 18 to a surface other than the syringe itself, such as a medical chart or a vial. A portion of the chart or vial, for example, with particularly sensitive information and which cannot and/or should not be obscured, may be placed under the under the window 36 where the information is clearly visible at all times despite the presence of the label 18. The window may also be placed over indicia or information which a user may want to highlight or, for example, bring to a doctor's attention.

There are a number of possible alternate embodiments for this invention. The above characterization of a particular exemplary embodiment is not intended to suggest that this invention is limited to the recited embodiment. While the above detailed description is limited to a syringe the present invention encompasses the use of label 18 on items other than a syringe, e.g., a vial and medical container. Any item with limited surface area available to print information may benefit from the use of the wrapped or rolled label of the present invention.

Those skilled in the art can appreciate from the foregoing description that the present invention can be implemented in a variety of forms. Therefore, while the embodiments of this invention have been described in connection with particular examples thereof, the true scope of the embodiments of the invention should not be so limited since other modifications and variations will become apparent to the skilled practitioner upon a study of the drawings and specification. Such modifications and variations are considered to be within the purview and scope of the appended claims and their equivalents.

## Claims

1. A syringe (10), comprising:
a barrel (12) having a flange (26) formed on one end and graduation markings (19) running along a longitudinal axis of the barrel configured to facilitate the administration of a metered dosage; and
a label (18) having a width and a length, the length being larger than the width and longer than a circumference of the barrel, a first end of the label connected to the barrel, such that the width of the label extends along the longitudinal axis, the label having a top surface and a bottom surface opposite the top surface facing the barrel when the label is wrapped around the barrel, at least a portion of the bottom surface exhibiting an adhesive property allowing the label to remain in at least a partially wrapped state around the barrel, wherein the label can be re-rolled around the barrel after being unrolled,
the label positioned directly below the flange between the graduation markings and the flange, and not covering the graduation markings even when the entire label is wrapped around the barrel, such that the label does not dangle from the syringe, wherein a circumference of the label wrapped around the barrel is sufficiently smaller than a circumference of the flange so as to allow a user to grasp the barrel over the wrapped label between the index and middle fingers without slipping over the flange while using the syringe.

2. The syringe of claim 1, wherein the graduation markings are at least one of (i) printed directly on the barrel, (ii) inscribed in the barrel, (iii) integrally formed with the barrel, and (iv) embossed in the barrel.

3. The syringe of claim 1, wherein at least a portion of the outer surface of the label, adjacent a second end of the label opposite the first end, is wrapped about the syringe, and has at least one of (i) a raised surface, and (ii) a high friction material adhered to the outer surface, configured to provide traction to prevent slippage of the user's fingers during use of the syringe.

4. The syringe of claim 1, wherein the label includes text on at least one of the bottom surface and the top surface, the label including a first portion, between the second end and a transition point on the label spaced away from the second end a length equal to a circumference of the barrel, and a second portion, between the transition point and the first end, the second portion covered by the first portion when the label is wrapped around the syringe and itself, text on the first portion oriented such that it runs along the width of the label and the longitudinal axis of the barrel, text on the second portion oriented such that it runs along a length of the label in a direction transverse to the longitudinal axis of the barrel.

5. The syringe of claim 4, wherein the text on the first portion includes a medication name and expiration date.

6. The syringe of claim 1, wherein the label is made from an electret material.

7. The syringe of claim 6, wherein the label includes an adhesive coating on the bottom surface.

8. The syringe of claim 6, wherein the electret material carries an electrical charge sufficient to maintain the label wrapped around the barrel and to cause the label to once again wrap itself about the barrel after a user unwraps the label from the barrel.

9. The syringe of claim 1, wherein the label has an adhesive coating on the bottom surface, and a pulling force is required to unwrap the label from the barrel and itself, wherein the adhesive coating is configured, such that, the further the label is unwrapped, progressively more pulling force is required to unwrap the label.

10. The syringe of claim 1, wherein the label has an adhesive coating on the bottom surface, and a pulling force is required to unwrap the label from the barrel and itself, wherein the adhesive coating is configured, such that, the further the label is unwrapped, progressively less pulling force is required to unwrap the label.

11. The syringe of claim 1, wherein the label has a plurality of spaced apart adhesive strips on the bottom surface and indicia on the bottom surface between the strips, different sections of label information included between each adhesive strip, wherein a user encounters an increased resistance to unwrapping the label upon the start of each section of label information.

12. The syringe of claim 1, wherein the label is folded along its length at least once prior to being connected to and wrapped around the barrel such that label in the unwrapped state can be unfolded by pulling a portion of the label along the longitudinal axis of the barrel.

## Patentansprüche

1. Spritze (10), umfassend:
eine Trommel (12) mit einem Flansch (26), der an einem Ende gebildet ist, und Einteilungsmarkierungen (19), die entlang einer Längsachse der Trommel verlaufen und dazu gestaltet sind, die Verabreichung einer abgemessenen Dosis zu erleichtern; und
ein Etikett (18) mit einer Breite und einer Länge, wobei die Länge länger als die Breite und länger als ein Umfang der Trommel ist, wobei ein erstes Ende des Etiketts so mit der Trommel verbunden ist, dass sich die Breite des Etiketts entlang der Längsachse erstreckt, wobei das Etikett eine obere Fläche und eine zu der oberen Fläche entgegengesetzte untere Fläche, die zu der Trommel gewandt ist, wenn das Etikett um die Trommel gewickelt ist, aufweist, wobei wenigstens ein Abschnitt der unteren Fläche Haftfähigkeit zeigt, wodurch dem Etikett gestattet wird, in einem wenigstens teilweise um die Trommel gewickelten Zustand zu bleiben, wobei das Etikett nach dem Abrollen wieder um die Trommel aufgerollt werden kann,
wobei das Etikett direkt unter dem Flansch zwischen den Einteilungsmarkierungen und dem Flansch positioniert ist und die Einteilungsmarkierungen auch dann nicht abdeckt, wenn das gesamte Etikett so um die Trommel gewickelt ist, dass das Etikett nicht von der Spritze hängt, wobei ein Umfang des Etiketts, das um die Trommel gewickelt ist, ausreichend kleiner als ein Umfang des Flanschs ist, um einem Benutzer zu gestatten, die Trommel über dem aufgewickelten Etikett zwischen dem Zeigefinger und dem Mittelfinger zu ergreifen, ohne über den Flansch zu rutschen, während die Spritze verwendet wird.

2. Spritze nach Anspruch 1, wobei die Einteilungsmarkierungen wenigstens eines aus (i) direkt auf die Trommel gedruckt, (ii) in die Trommel graviert, (iii) einstückig mit der Trommel gebildet, oder (iv) in die Trommel geprägt sind.

3. Spritze nach Anspruch 1, wobei wenigstens ein Teil der Außenfläche des Etiketts neben einem zweiten Ende des Etiketts, das zu dem ersten Ende entgegengesetzt ist, um die Spritze gewickelt ist und wenigstens eines aus (i) einer erhöhten Oberfläche, und (ii) einem an die Außenfläche geklebten Material mit hoher Reibung aufweist, die bzw. das zur Bereitstellung von Haftung gestaltet ist, um ein Rutschen der Finger des Benutzers während der Verwendung der Spritze zu verhindern.

4. Spritze nach Anspruch 1, wobei das Etikett Text auf wenigstens einem aus der unteren Fläche und der oberen Fläche umfasst, wobei das Etikett einen ersten Abschnitt zwischen dem zweiten Ende und einem Übergangspunkt auf dem Etikett, der von dem zweiten Ende um eine Länge beabstandet ist, die dem Umfang der Trommel gleich ist, und einen zweiten Abschnitt zwischen dem Übergangspunkt und dem ersten Ende umfasst, wobei der zweite Abschnitt von dem ersten Abschnitt bedeckt ist, wenn das Etikett um die Spritze und sich selbst gewickelt ist, wobei Text auf dem ersten Abschnitt so ausgerichtet ist, dass er entlang der Breite des Etiketts und der Längsachse der Trommel verläuft, und Text auf dem zweiten Abschnitt so ausgerichtet ist, dass er entlang einer Länge des Etiketts in eine quer zu der Längsachse der Trommel verlaufende Richtung verläuft.

5. Spritze nach Anspruch 4, wobei der Text auf dem ersten Abschnitt eine Medikamentenbezeichnung und ein Verfallsdatum enthält.

6. Spritze nach Anspruch 1, wobei das Etikett aus einem Elektret-Material besteht.

7. Spritze nach Anspruch 6, wobei das Etikett eine Klebebeschichtung an der unteren Fläche umfasst.

8. Spritze nach Anspruch 6, wobei das Elektret-Material eine elektrische Ladung trägt, die ausreichend ist, um das Etikett um die Trommel gewickelt zu halten, und zu verursachen, dass sich das Etikett erneut selbst um die Trommel wickelt, sobald ein Benutzer das Etikett von der Trommel abwickelt.

9. Spritze nach Anspruch 1, wobei das Etikett eine Klebebeschichtung an der unteren Fläche aufweist, und eine Zugkraft erforderlich ist, um das Etikett von der Trommel und sich selbst abzuwickeln, wobei die Klebebeschichtung so gestaltet ist, dass progressiv umso mehr Zugkraft erforderlich ist, um das Etikett abzuwickeln, je weiter das Etikett abgewickelt ist.

10. Spritze nach Anspruch 1, wobei das Etikett eine Klebebeschichtung an der unteren Fläche aufweist, und eine Zugkraft erforderlich ist, um das Etikett von der Trommel und sich selbst abzuwickeln, wobei die Klebebeschichtung so gestaltet ist, dass progressiv um so weniger Zugkraft erforderlich ist, um das Etikett abzuwickeln, je weiter das Etikett abgewickelt ist.

11. Spritze nach Anspruch 1, wobei das Etikett an der unteren Fläche mehrere voneinander beabstandete Klebestreifen und Markierungen an der unteren Fläche zwischen den Klebestreifen aufweist, wobei zwischen den einzelnen Klebestreifen unterschiedliche Abschnitte von Etiketteninformationen enthalten sind, wobei ein Benutzer am Beginn jedes Abschnitts von Etiketteninformationen auf einen zunehmenden Widerstand gegen das Abwickeln des Etiketts stößt.

12. Spritze nach Anspruch 1, wobei das Etikett vor seinem Verbinden mit und seinem Wickeln um die Trommel wenigstens ein Mal so entlang seiner Länge gefaltet ist, dass das Etikett in dem abgewickelten Zustand durch Ziehen eines Teils des Etiketts entlang der Längsachse der Trommel entfaltet werden kann.

## Revendications

1. Seringue (10), comprenant:
un cylindre (12) comprenant une bride (26) formée sur une extrémité, et des marques de graduation (19) qui s'étendent le long d'un axe longitudinal du cylindre et sont prévues dans le but de faciliter l'administration d'une quantité dosée; et
une étiquette (18) présentant une largeur et une longueur, la longueur étant plus grande que la largeur et plus grande qu'une circonférence du cylindre, une première extrémité de l'étiquette étant connectée au cylindre, de telle sorte que la largeur de l'étiquette s'étende le long de l'axe longitudinal, l'étiquette présentant une surface supérieure et une surface inférieure opposée à la surface supérieure qui fait face au cylindre lorsque l'étiquette est enroulée autour du cylindre, au moins une partie de la surface inférieure présentant une propriété adhésive qui permet à l'étiquette de rester dans un état au moins partiellement enroulé autour du cylindre, dans laquelle l'étiquette peut être ré-enroulée autour du cylindre après en avoir été déroulée,
l'étiquette étant positionnée directement en dessous de la bride entre les marques de graduation et la bride, et ne recouvrant pas les marques de graduation même lorsque la totalité de l'étiquette est enroulée autour du cylindre, de telle sorte que l'étiquette ne pende pas de la seringue, dans laquelle une circonférence de l'étiquette enroulée autour du cylindre soit suffisamment plus petite qu'une circonférence de la bride pour permettre à un utilisateur de saisir le cylindre sur l'étiquette enroulée entre l'index et le majeur sans glisser sur la bride lorsqu'il utilise la seringue.

2. Seringue selon la revendication 1, dans laquelle les marques de graduation sont au moins soit (i) imprimée directement sur le cylindre, soit (ii) inscrites dans le cylindre, soit (iii) formées intégralement avec le cylindre, soit (iv) estampées dans le cylindre.

3. Seringue selon la revendication 1, dans laquelle au moins une partie de la surface extérieure de l'étiquette, adjacente à une deuxième extrémité de l'étiquette opposée à la première extrémité, est enroulée autour de la seringue, et présente au moins soit (i) une surface en relief, soit (ii) un matériau à coefficient de friction élevé qui est collé sur la surface extérieure, configuré pour assurer une traction afin d'empêcher le glissement des doigts de l'utilisateur pendant l'utilisation de la seringue.

4. Seringue selon la revendication 1, dans laquelle l'étiquette comporte du texte sur au moins soit la surface inférieure, soit la surface supérieure, l'étiquette comprenant une première partie, entre la deuxième extrémité et un point de transition sur l'étiquette espacé de la deuxième extrémité d'une longueur égale à la circonférence du cylindre, et une deuxième partie, entre le point de transition et la première extrémité, la deuxième partie étant couverte par la première partie lorsque l'étiquette est enroulée autour de la seringue et sur elle-même, le texte sur la première partie étant orienté de telle sorte qu'il s'étende le long de la largeur de l'étiquette et de l'axe longitudinal du cylindre, le texte sur la deuxième partie étant orienté de telle sorte qu'il s'étende le long d'une longueur de l'étiquette dans une direction transversale à l'axe longitudinal du cylindre.

5. Seringue selon la revendication 4, dans laquelle le texte sur la première partie comprend un nom de médicament et une date d'expiration.

6. Seringue selon la revendication 1, dans laquelle l'étiquette est constituée d'une matière d'électret.

7. Seringue selon la revendication 6, dans laquelle l'étiquette comprend un revêtement adhésif sur la surface inférieure.

8. Seringue selon la revendication 6, dans laquelle la matière d'électret porte une charge électrique suffisante pour maintenir l'étiquette enroulée autour du cylindre et pour entraîner l'étiquette à s'enrouler une nouvelle fois sur elle-même autour du cylindre après qu'un utilisateur ait déroulé l'étiquette du cylindre.

9. Seringue selon la revendication 1, dans laquelle l'étiquette comporte un revêtement adhésif sur la surface inférieure, et une force de traction est requise pour dérouler l'étiquette du cylindre et d'elle-même, dans laquelle le revêtement adhésif est configuré de telle sorte que, plus l'étiquette est déroulée, progressivement plus de force de traction est nécessaire pour dérouler l'étiquette.

10. Seringue selon la revendication 1, dans laquelle l'étiquette comporte un revêtement adhésif sur la surface inférieure, et une force de traction est requise pour dérouler l'étiquette du cylindre et d'elle-même, dans laquelle le revêtement adhésif est configuré de telle sorte que, plus l'étiquette est déroulée, progressivement moins de force de traction est nécessaire pour dérouler l'étiquette.

11. Seringue selon la revendication 1, dans laquelle l'étiquette comprend une pluralité de bandes adhésives espacées les unes des autres sur la surface inférieure, et des repères sur la surface inférieure entre les bandes, différentes sections d'informations d'étiquette étant prévues entre chaque bande adhésive, dans laquelle un utilisateur rencontre une résistance accrue pour dérouler l'étiquette au début de chaque section d'informations d'étiquette.

12. Seringue selon la revendication 1, dans laquelle l'étiquette est pliée le long de sa longueur au moins une fois avant d'être connectée et enroulée autour du cylindre, de telle sorte que l'étiquette, dans l'état déroulé, puisse être dépliée en tirant sur une partie de l'étiquette le long de l'axe longitudinal du cylindre.
